# FASCICULE DE BREVET EUROPEEN

(11) **EP 1 758 630 B1**
(45) Date de publication et mention de la délivrance du brevet: **23.12.2009**
(21) Numéro de dépôt: 05777251.9
(22) Date de dépôt: 15.06.2005
(51) Int. Cl.: A61M 5/50, A61M 5/32

(54) **DISPOSITIF D'INJECTION A USAGE UNIQUE D'UN FLUIDE MEDICAMENTEUX**
EINWEGVORRICHTUNG ZUM INJIZIEREN VON FLÜSSIGEN ARZNEIMITTELN
SINGLE-USE DEVICE FOR INJECTING A MEDICAMENT FLUID

(30) Priorité: 18.06.2004 FR 0406618
(43) Date de publication de la demande: 07.03.2007
(73) Titulaire: MB Innovation, 31000 Toulouse (FR)
(72) Inventeur: BRUNEL, Marc, F-31000 Toulouse (FR); CANY, Lucien, F-81660 Pont de L'arn (FR)
(74) Mandataire: Morelle, Guy Georges Alain
(86) Numéro de dépôt international: PCT/FR2005/001487
(87) Numéro de publication internationale: WO 2006/008366

(56) Documents cités:
- EP-A- 0 680 767
- FR-A- 2 807 665
- FR-A- 2 830 765
- US-A1- 2003 014 018
- US-B1- 6 319 234

## Description

L'invention concerne un dispositif d'injection à usage unique doté d'un dispositif de protection après usage de l'aiguille d'injection, et vise plus spécifiquement de tels dispositifs d'injection dotés d'un corps de seringue comportant des organes de prise digitale d'un seul tenant avec le dit corps de seringue.

A l'heure actuelle, pour des raisons évidentes de sécurité, les dispositifs d'injection à usage unique sont couramment équipés de dispositifs de protection après usage de l'aiguille d'injection, conçus, d'une part, pour permettre d'injecter la dose de produit médicamenteux selon une méthodologie classique, puis, d'autre part, pour protéger l'aiguille d'injection après injection, et interdire ensuite toute réutilisation ultérieure du dispositif d 'injection.

A cet effet, de tels dispositifs d'injection comprennent :
- un corps de seringue comportant, d'une part, une embase portant une aiguille d'injection dotée d'une partie active d'injection dans le prolongement de la dite embase, et, d'autre part, à l'opposé de cette embase, des organes de prise digitale d'un seul tenant avec le dit corps de seringue,
- un capuchon protége-aiguille de forme adaptée pour loger la partie active de l'aiguille d'injection, et coiffer l'embase du corps de seringue,
- un étui tubulaire de protection après usage de l'aiguille d'injection, de section interne adaptée pour coulisser extérieurement le long du corps de seringue,
- les dits corps de seringue et étui de protection étant dotés d'organes de blocage relatif en translation consistant en :
   des premiers organes de blocage disposés et adaptés pour bloquer l'étui de protection dans une position reculée, dite position d'injection, dans laquelle le dit étui de protection se trouve escamoté longitudinalement par rapport à la partie active de l'aiguille d'injection,

   - et des seconds organes de blocage ménagés sur le corps de seringue et à l'intérieur de l'étui de protection, adaptés pour bloquer de façon irréversible l'étui de protection dans une position avancée de protection après usage de l'aiguille d'injection, dans laquelle le dit étui de protection s'étend partiellement dans le prolongement du corps de seringue de façon à loger la partie active de la dite aiguille d'injection,
   - les dits seconds organes de blocage ménagés sur le corps de seringue présentant une section externe adaptée pour coulisser à l'intérieur de l'étui de protection, et formant, sur le pourtour du dit corps de seringue, deux faces radiales annulaires, dites face de butée postérieure et face de butée antérieure,
   - et les dits seconds organes de blocage ménagés à l'intérieur de l'étui de protection formant deux faces radiales de butée s'étendant en regard l'une de l'autre, ménagées de façon à coopérer chacune avec une des faces de butée radiale formées sur le pourtour du corps de seringue.

Selon ce principe, compte tenu de la présence des organes de prise digitale solidaires du corps de seringue, l'assemblage du dispositif d'injection impose de présenter l'embase du corps de seringue en regard de l'extrémité postérieure de l'étui de protection, puis de faire pénétrer le dit corps de seringue dans le dit étui de protection jusqu'à obtenir un positionnement relatif de ces éléments correspondant à la position d'injection du dispositif d'injection.

La principale difficulté rencontrée lors de cet assemblage réside dans le fait que, lors de leur coulissement relatif, l'étui de protection et le corps de seringue sont amenés, en premier lieu, dans leur position avancée de protection après usage de l'aiguille d'injection, puis doivent franchir cette position avancée pour être amenés dans leur position reculée d'injection.

Or, les seconds organes de blocage possèdent des faces radiales de butée conçues pour bloquer de façon irréversible l'étui de protection dans sa position avancée, et donc, autrement dit, conçues pour interdire le franchissement précité autorisant un recul de cet étui de protection vers la position d'injection du dispositif d'injection.

Pour cette raison, un artifice de fabrication doit être mis au point pour permettre de supprimer, lors de l'assemblage du dispositif d'injection, le caractère irréversible du blocage de l'étui de protection dans sa position avancée.

A l'heure actuelle, diverses solutions ont été proposées, qui consistent toutes à résoudre le problème posé par des aménagements concernant soit le corps de seringue, soit l'étui de protection.

A titre d'exemple, on peut ainsi citer, concernant une solution visant un aménagement du corps de seringue, la solution décrite dans EP740942 qui consiste à réaliser un élément de corps de seringue doté d'un tronçon déformable radialement lors de l'assemblage, au niveau duquel sont ménagés les seconds organes de blocage.

. Comme solution visant un aménagement de l'étui de protection, on peut citer la solution décrite dans WO 01/24855 ou EP 0 680 767 qui consiste à réaliser un étui de protection très spécifique constitué de deux corps, postérieur et antérieur, aptes à être assemblés dans le prolongement l'un de l'autre.

Toutes les solutions proposées actuellement permettent de solutionner le problème technique posé. Toutefois, elles imposent des aménagements du corps de seringue et/ou de l'étui de protection qui, d'une part, conduisent à des surcoûts de production, et d'autre part, s'avèrent imposer de réaliser, pour chaque solution, un couple spécifique étui de protection/corps de seringue de formes complémentaires.

La présente invention vise à pallier ces inconvénients et a pour principal objectif de fournir un dispositif d'injection du type ci-dessus décrit, dont l'assemblage ne requiert aucun aménagement spécifique du corps de seringue et de l'étui de protection, et ne conduit à aucun surcoût de production.

Un autre objectif de l'invention est de fournir une solution pouvant s'adapter de façon universelle à tout type d'étui de protection et de corps de seringue.

A cet effet, le dispositif d'injection selon l'invention se **caractérise en ce que** le capuchon protége-aiguille comporte longitudinalement un tronçon antérieur de longueur adaptée pour loger au moins partiellement la partie active de l'aiguille d'injection, et un tronçon postérieur relié au dit tronçon antérieur par un tronçon intermédiaire de forme tronconique s'évasant en direction du dit tronçon postérieur :
- le dit capuchon protége-aiguille présentant une longueur totale adaptée pour que l'extrémité postérieure du tronçon postérieur vienne buter contre la face de butée antérieure des seconds organes de blocage ménagés sur le corps de seringue,
- et le dit tronçon postérieur présentant un contour conjugué de celui de la face de butée antérieure précitée, de façon à se profiler extérieurement avec cette dernière, et à la masquer.

Selon le principe de l'invention, lors de l'assemblage du corps de seringue et de l'étui de protection, la face de butée antérieure des seconds organes de blocage ménagés sur le corps de seringue est masquée par le tronçon postérieur du capuchon protége-aiguille, qui se profile et s'étend dans le prolongement longitudinal de cette face de butée.

Ainsi, lors du coulissement relatif entre l'étui de protection et le corps de seringue conduisant à l'assemblage de ces deux éléments, aucun élément du corps de seringue ne forme une butée orthogonale à l'axe de déplacement longitudinal de l'étui de protection, susceptible de constituer un obstacle s'opposant à ce coulissement relatif.

Au contraire, ce coulissement relatif est facilité par la forme tronconique du tronçon intermédiaire du capuchon protége-aiguille qui forme une rampe longitudinale le long de laquelle l'étui de protection est amené à se déplacer en subissant une expansion radiale autorisée classiquement par la capacité de déformation élastique que possèdent les matériaux conventionnels de réalisation des étuis de protection, ce qui permet, par la suite, à cet étui de protection, de pouvoir coulisser aisément le long du tronçon postérieur du capuchon protége-aiguille et de parvenir, au final, dans sa position reculée d'injection.

Le franchissement par l'étui de protection des seconds organes de blocage ménagés sur le corps de seringue, résulte, donc, selon l'invention, du profil longitudinal spécifique que possède le capuchon protége-aiguille ; ce franchissement est donc autorisé grâce à la conception d'un élément amovible destiné à être retiré en vue de l'injection, de façon à restituer aux seconds organes de blocage leur fonction de blocage irréversible de l'étui de protection dans sa position de protection après usage de l'aiguille d'injection.

Le surcoût de production que génère la solution technique selon l'invention est donc négligeable. De plus, cette solution technique ne nécessite aucune adaptation ni du corps de seringue ni de l'étui de protection, de sorte qu'elle peut s'adapter de façon universelle à tout type d'étui de protection et de corps de seringue.

Selon un mode de réalisation avantageux visant un dispositif d'injection doté d'un étui de protection à déclenchement manuel après injection, les premiers organes de blocage relatif en translation comprennent une nervure interne de section non rétentive ménagée au niveau d'une portion de tronçon antérieur du dit étui de protection, et adaptée pour coopérer avec la face de butée postérieure des seconds organes de blocage ménagés sur le corps de seringue.

Selon un autre mode réalisation avantageux de l'invention, le corps de seringue comporte un récipient tubulaire prolongé d'un raccord conique mâle, et une embase formant un raccord conique femelle conjugué, et sur laquelle sont ménagés les seconds organes de blocage relatif en translation.

Selon une variante de réalisation avantageuse de l'invention, le corps de seringue comporte, d'un seul tenant, un récipient tubulaire prolongé d'une embase porte-aiguille d'injection, et sur lequel sont ménagés, sensiblement en retrait longitudinal par rapport à la dite embase, les seconds organes de blocage relatif en translation.

Selon un autre mode de réalisation avantageux de l'invention, les seconds organes de blocage ménagés sur le corps de seringue consistent en une nervure annulaire dotée d'une paroi périphérique circulaire de diamètre adapté pour former un appui de guidage du coulissement de l'étui de protection, et de deux parois frontales radiales formant les faces postérieure et antérieure de butée.

Par ailleurs, le principe de l'invention s'applique à toutes les dimensions de dispositifs d'injection, et peut donc notamment être mis en oeuvre lorsque les corps de seringue présentent un diamètre susceptible d'autoriser à une personne d'introduire un doigt à l'intérieur de l'étui de protection.

Dans ce cas là, en vue de se garantir contre tout risque d'accident par piqûre après injection, et de façon avantageuse selon l'invention, l'étui tubulaire comporte un tronçon antérieur de section interne décroissant longitudinalement, de sorte que le diamètre de l'extrémité de l'étui de protection est ramené à une valeur garantissant contre toute introduction d'un doigt dans le dit étui. De plus, selon l'invention :
- le capuchon protége-aiguille se compose de deux éléments distinctes aptes à être assemblés dans le prolongement l'un de l'autre:
   ■ un élément antérieur formant le tronçon antérieur du dit capuchon protége-aiguille, et adapté pour coiffer l'embase du corps de seringue,
   ■ et un élément postérieur formant les tronçons intermédiaire et postérieur du capuchon protége-aiguille, et adapté pour être logé à l'intérieur de l'étui de protection dans la position reculée d'injection de ce dernier,
   ■ l'élément postérieur étant adapté, après retrait de l'élément antérieur, pour être entraîné avec l'étui de protection lors du déplacement de ce dernier vers sa position de protection après usage de l'aiguille d'injection.

En vue d'entraîner l'élément postérieur du capuchon protége-aiguille avec l'étui de protection, et selon une première variante avantageuse de l'invention, le dit élément postérieur est conformé de façon à constituer un tronçon intermédiaire adapté pour se bloquer par un effet de coincement à l'intérieur du tronçon antérieur du dit étui de protection, dans la position d'injection de ce dernier.

Selon une deuxième variante avantageuse de l'invention visant à fournir un dispositif d'injection à « déclenchement automatique » de l'étui de protection après injection, l'élément postérieur du capuchon protége-aiguille délimite, autour de l'embase du corps de seringue, un volume annulaire apte à loger dans son état comprimé un ressort de propulsion adapté pour entraîner, après déclenchement, un déplacement simultané du dit élément postérieur et de l'étui de protection.

D'autres caractéristiques, buts et avantages de l'invention ressortiront de la description détaillée qui suit en référence aux dessins annexés qui en représentent à titre d'exemples non limitatifs quatre modes de réalisation préférentiels. Sur ces dessins :

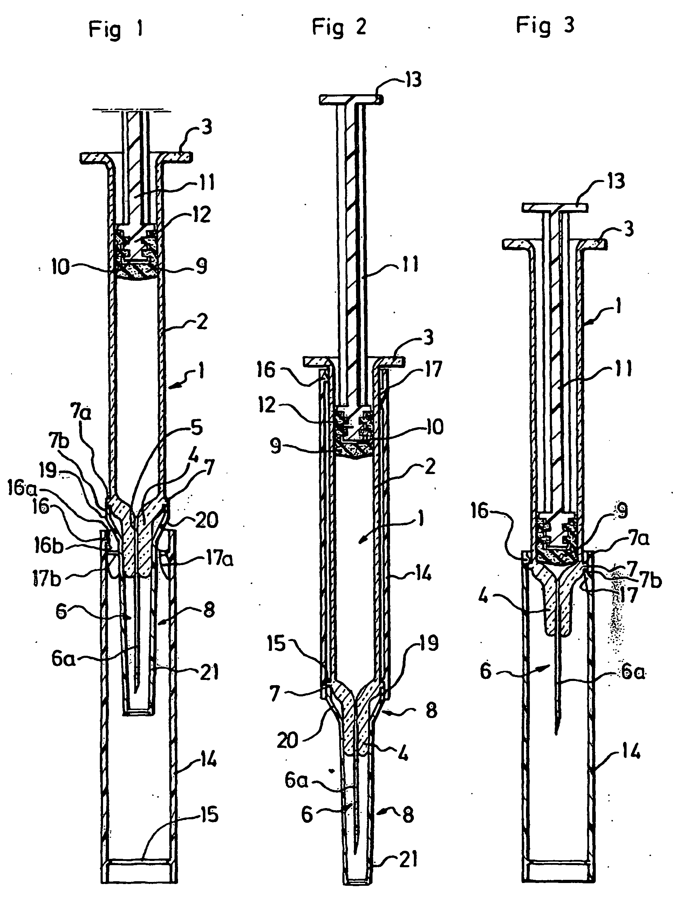

- les figures 1 à 3 sont des coupes longitudinales par un plan axial d'un premier mode de réalisation de dispositif d'injection selon l'invention représenté respectivement :
   ■ figure 1, lors de la phase d'assemblage du corps de seringue et de l'étui de protection,
   ■ figure 2, dans la position reculée d'injection de l'étui de protection,
   ■ figure 3, dans la position de protection après usage de l'étui de protection,

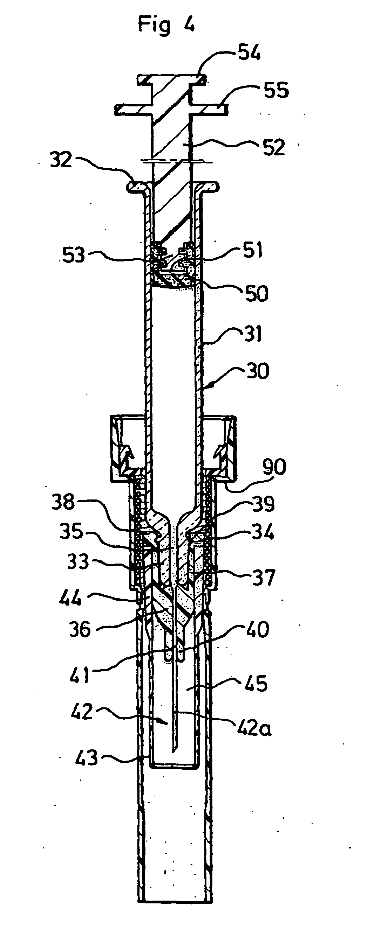

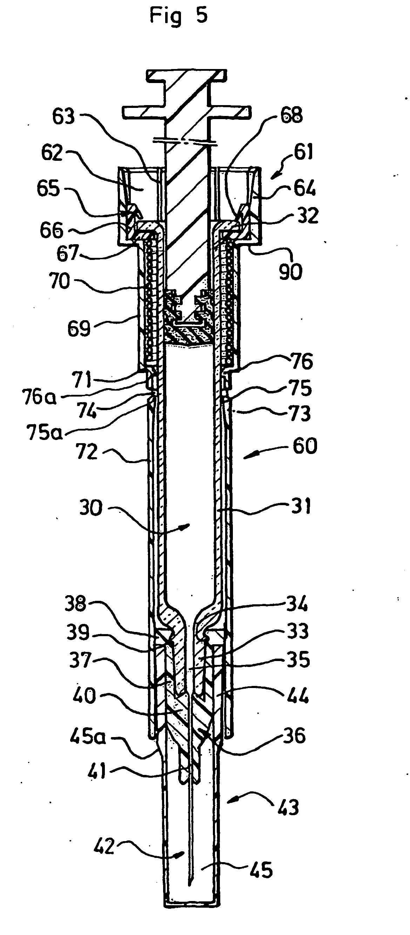

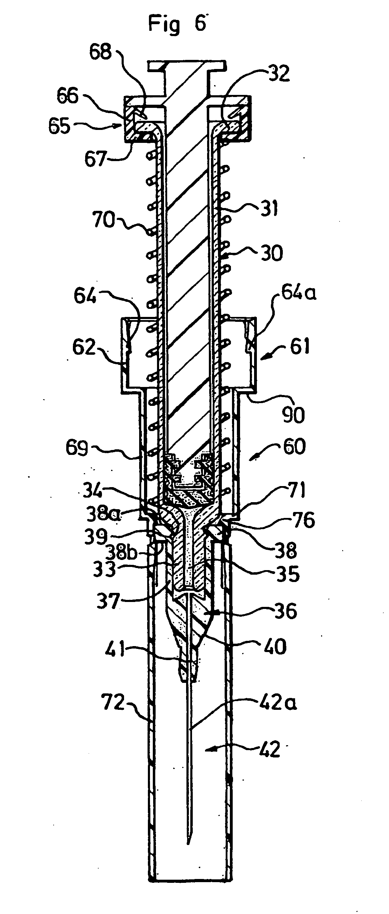

- les figures 4 à 6 sont des coupes longitudinales par un plan axial d'un deuxième mode de réalisation de dispositif d'injection selon l'invention représenté également, de façon respective, lors de sa phase d'assemblage (figure 4), dans la position reculée d'injection de l'étui de protection (figure 5), et dans la position de protection après usage de l'étui de protection (figure 6),

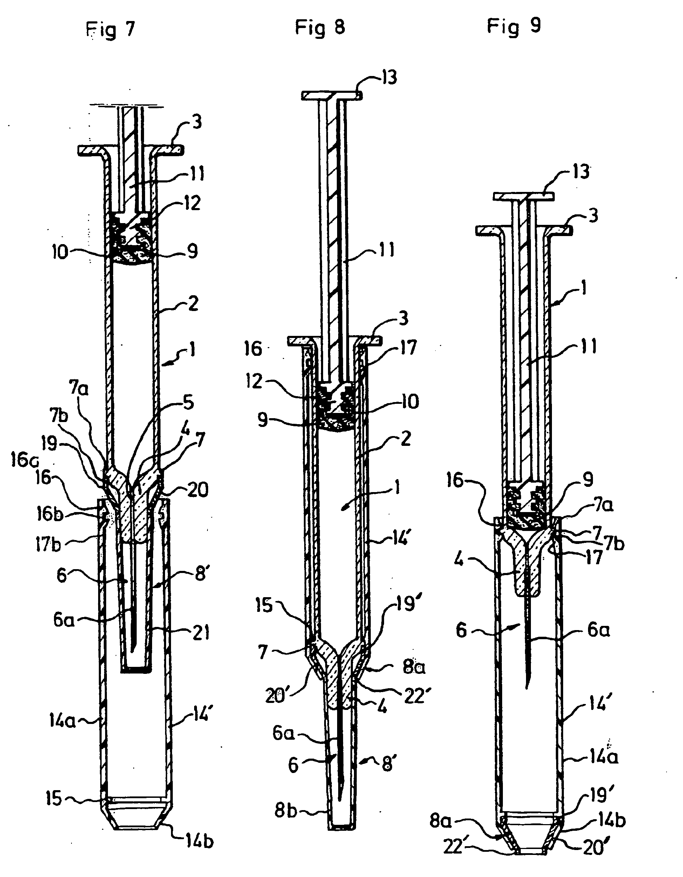

- les figures 7 à 9 sont des coupes longitudinales par un plan axial d'un troisième mode de réalisation de dispositif d'injection selon l'invention représenté, de façon respective, lors de sa phase d'assemblage (figure 7), dans la position reculée d'injection de l'étui de protection (figure 8), et dans la position de protection après usage de l'étui de protection (figure 9),

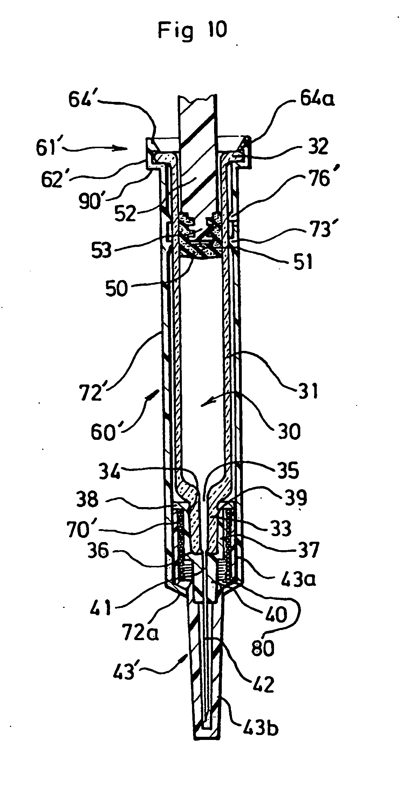

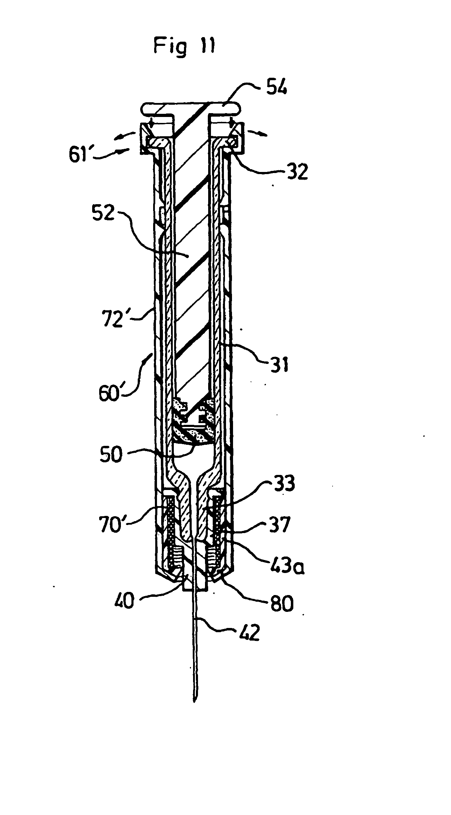

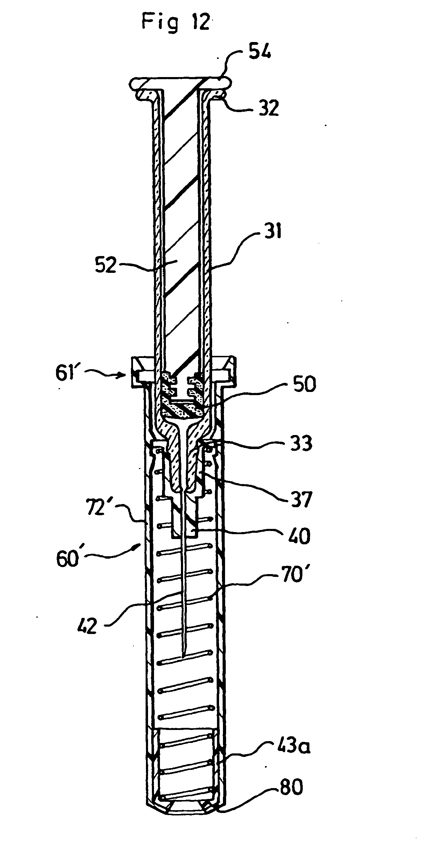

- et les figures 10 à 12 sont des coupes longitudinales par un plan axial d'un quatrième mode de réalisation de dispositif d'injection selon l'invention représenté également, de façon respective, lors de sa phase d'assemblage (figure 10), dans la position reculée d'injection de l'étui de protection (figure 11), et dans la position de protection après usage de l'étui de protection (figure 12)

Les figures 1-3, 4-6, 7-9, et 10-12 représentent respectivement quatre variantes de réalisation de dispositifs d'injection à usage unique selon l'invention, comportant un corps de seringue pré-rempli d'une dose de médicament à injecter, et un étui de protection après usage de l'aiguille d'injection, apte, en outre, à interdire toute réutilisation du dit dispositif d'injection.

En premier lieu, les figures 1-3 et 4-6 représentent deux dispositifs d'injection dont les corps de seringue présentent un diamètre d'une valeur non susceptible d'autoriser à une personne d'introduire un doigt à l'intérieur de l'étui de protection.

De plus, selon les figures 1 à 3, le dispositif d'injection est du type à « déclenchement manuel » de l'étui de protection après injection, tandis que, selon les figures 4 à 6, le dispositif d'injection est du type à « déclenchement automatique » de l'étui de protection après injection.

Selon les figures 1 à 3, le corps de seringue 1 du dispositif d'injection consiste en une seringue classique, du type seringue en verre conventionnelle, formée d'un tube cylindrique 2 doté d'une collerette appui-doigts 3 au niveau de son extrémité postérieure, et prolongé, au niveau de son extrémité antérieure, par une embase tronconique 4. De plus, de façon classique, l'embase 4 est percée axialement d'un conduit longitudinal 5 d'alimentation d'une aiguille d'injection 6 scellée dans cette embase 4 de façon à présenter un tronçon actif d'injection 6a dans le prolongement de la dite embase.

Cette seringue 1 comporte, en outre, une nervure externe 7 formée sur le pourtour du tube cylindrique 2, légèrement en amont de la jonction de ce dernier avec l'embase 4, la dite nervure présentant une forme annulaire délimitée par une face périphérique cylindrique et deux faces frontales radiales respectivement postérieure 7a et antérieure 7b.

Le dispositif d'injection selon les figures 1 à 3 comprend également un capuchon protège-aiguille 8, décrit en détail plus loin, de forme adaptée pour loger la partie active 6a de l'aiguille d'injection 6, et coiffer l'embase 4 du corps de seringue 1.

Ce dispositif d'injection comporte, en outre, de façon classique, un piston 9 de forme adaptée pour coulisser de façon étanche dans le tube cylindrique 2, percé d'un alésage borgne taraudé 10 conjugué de l'extrémité antérieure filetée 12 d'une tige de piston 11 dotée, au niveau de son extrémité opposée, d'un poussoir 13 d'actionnement digital du piston 9.

Le dispositif d'injection comprend également un étui de protection 14 de forme cylindrique adaptée pour coulisser le long du corps de seringue 1, et réalisé par exemple par moulage par injection d'un matériau plastique. Cet étui de protection 14 comporte, en premier lieu, une nervure annulaire interne 15 de forme non rétentive, ménagée à faible distance de l'extrémité antérieure du dit étui de protection 1.

Cet étui de protection 14 comporte également des organes de blocage internes ménagés au niveau de son extrémité postérieure, formés de deux séries de crans, dits postérieurs, tels que 16, et antérieurs, tels que 17, espacées axialement l'une de l 'autre, de façon à délimiter une gorge annulaire de forme rétentive apte à loger la nervure annulaire 7 du corps de seringue 1.

En vue de conférer son caractère rétentif à la gorge, chacun de ces crans 16, 17 présente la forme d'une dent asymétrique, et les crans postérieurs 16 sont disposés longitudinalement en position inversée par rapport aux crans antérieurs 17, de façon que :
. les crans postérieurs 16 présentent une face postérieure 16a à profil oblique formant une rampe, et une face antérieure radiale 16b de butée mécanique,
les crans antérieurs 17 présentent une face antérieure 17a à profil oblique formant une rampe, et une face postérieure radiale 17b de butée mécanique.

De plus, les crans 16, 17 de chacune de ces séries sont uniformément répartis autour de l'axe longitudinal de l'étui de protection 14, et les crans de chaque série sont décalés angulairemeni par rapport à ceux de l'autre série.

En dernier lieu, et selon l'invention, le capuchon protége-aiguille 8 de ce dispositif d'injection se compose longitudinalement de trois tronçons consistant en :
. un tronçon antérieur 21 de forme et de longueur adaptées pour loger la partie active 6a de l'aiguille d'injection 6, et pour coiffer l'embase 4,
. un tronçon postérieur cylindrique 19 de faible longueur, de diamètre interne sensiblement supérieur au diamètre externe du tube cylindrique 2 du corps de seringue 1, et de diamètre externe égal au diamètre externe de la nervure annulaire 7,
. et un tronçon intermédiaire tronconique 20 de liaison entre les tronçons postérieur 19 et antérieur 21.

De plus, et de façon essentielle selon l'invention, les dimensions de ces trois tronçons 19-21 sont adaptées pour que, lors du montage du capuchon 8 sur le corps de seringue 1 :
. le tronçon antérieur 21 vienne se bloquer par un effet de coincement sur l'embase 4 du corps de seringue 1,
. et le tronçon postérieur 19 vienne buter contre la face antérieure radiale 7b de la nervure 7, de façon que le dit tronçon postérieur se profile dans le prolongement de la dite nervure.

Selon ce principe, et tel que notamment représenté à la figure 1, lors de l'assemblage du corps de seringue 1 et de l'étui de protection 14, l'extrémité postérieure puis les crans postérieurs 16, et enfin les crans antérieurs 17 du dit étui viennent, en premier lieu, en contact avec le tronçon intermédiaire 20 du capuchon protége-aiguille 8, le long duquel les dits crans sont amenés à coulisser en subissant une expansion radiale autorisée par la capacité de déformation élastique que possède classiquement la matière plastique conventionnellement utilisée pour la réalisation par injection des étuis de protection.

Dans la continuité du déplacement relatif de l'étui de protection 14 et du corps de seringue 1, les crans 16, 17 sont ensuite amenés à coulisser le long du tronçon postérieur19 du capuchon protégé-aiguille 8, puis le long de la face périphérique de la nervure 7 du corps de seringue 1, et enfin le long du tube cylindrique 2, jusqu'à parvenir dans la position reculée d'injection du dit étui, représentée à la figure 2, dans laquelle un blocage relatif en translation réversible est obtenu par butée de la nervure interne15 de l'étui de protection 14 contre la face postérieure radiale 7a de la nervure 7 du corps de seringue 1.

Lors de ce coulissement relatif, aucun élément du corps de seringue 1 ne forme une butée orthogonale à l'axe de déplacement longitudinal de l'étui de protection 14, susceptible de constituer un obstacle s'opposant à ce coulissement relatif, de sorte que l'assemblage peut être réalisé de façon classique jusqu'à obtenir la position d'injection de l'étui de protection 14.

Par contre, après retrait du capuchon protége-aiguille 8 en vue d'une injection, la face antérieure 7b de la nervure 7 du corps de seringue 1 se trouve « démasquée », de sorte, qu'après injection et tel que représenté à la figure 3, cette face antérieure 7b forme une butée radiale pour la face postérieure 17b des crans 17 de l'étui de protection 14, provoquant un blocage irréversible de ce dernier dans sa position de protection après usage de l'aiguille d'injection 6.

Selon les figures 4 à 6, le dispositif d'injection est du type à « déclenchement automatique » de l'étui de protection après injection.

Le corps de seringue 30 de ce dispositif d'injection comporte une seringue formée d'un tube cylindrique 31 doté d'une collerette appui-doigts 32 au niveau de son extrémité postérieure, et prolongé, au niveau de son extrémité antérieure, par un nez tronconique 33 formant un raccord mâle à verrouillage percé longitudinalement d'un alésage axial 35. De plus, ce nez 33 présente une nervure externe annulaire 34 formée sensiblement en aval de la jonction du dit nez avec le tube cylindrique 31.

Ce corps de seringue 30 comporte, en outre, une embase 36 formant un raccord conique femelle 37 à verrouillage conjugué du raccord mâle que constitue le nez 33, et à l'intérieur duquel est formée une rainure annulaire 39 apte à venir s'encliqueter sur la nervure 34 du nez 33, lors de l'assemblage entre les dits nez et embase.

Cette embase 36 comporte également, dans le prolongement du raccord femelle 37, un col 40 percé d'un alésage axial 41 dans lequel est scellé le tronçon postérieur d'une aiguille d'injection 42 adaptée pour présenter un tronçon actif d'injection 42a dans le prolongement du dit col.

Cette embase 36 comporte, enfin, une nervure externe 38 formée sur le pourtour du raccord femelle 37, au niveau de l'extrémité postérieure du dit raccord, la dite nervure présentant une forme annulaire délimitée par une face périphérique cylindrique et deux faces frontales radiales respectivement postérieure 38a et antérieure 38b.

Le dispositif d'injection selon les figures 4 à 6 comprend également un capuchon protége-aiguille 43 de forme adaptée pour loger la partie active 42a de l'aiguille d'injection 42, coiffer l'embase 36 du corps de seringue 30 et se bloquer par un effet de coincement sur cette dernière, et venir en butée contre la face radiale antérieure 38b de la nervure 38 de la dite embase.

Ce capuchon protége-aiguille 43, de forme générale cylindrique, comporte trois tronçons longitudinaux de même diamètre interne et de diamètres externes différents obtenus par des variations de l'épaisseur de la paroi périphérique du dit capuchon, et consistant en :
. un tronçon postérieur 44 de diamètre externe constant égal au diamètre externe de la nervure 38 de l'embase 36, de façon que le dit tronçon postérieur se profile dans le prolongement de la dite nervure,
. un tronçon antérieur 45 de diamètre externe constant et inférieur au diamètre externe du tronçon postérieur 44,
. et un tronçon intermédiaire 45a de liaison des tronçons postérieur 44 et antérieur 45, formant un épaulement externe de forme longitudinale tronconique.

Ce dispositif d'injection comporte, en outre, de façon classique, un piston 50 de forme adaptée pour coulisser de façon étanche dans le tube cylindrique 31, percé d'un alésage borgne taraudé 51 conjugué de l'extrémité antérieure filetée 53 d'une tige de piston 52 dotée, au niveau de son extrémité opposée, d'un poussoir 54 d'actionnement digital du piston 50.

Cette tige de piston 52 comporte, en outre, à faible distance de son extrémité postérieure, un disque 55 de diamètre sensiblement supérieur à celui de la collerette appui-doigts 32, adapté, tel que décrit plus loin, pour engendrer de façon automatique, en fin d'injection, le déclenchement du déplacement de l'étui de protection 60 vers sa position de protection après usage.

Cet étui de protection 60 se subdivise longitudinalement en trois tronçons principaux de formes cylindriques et de diamètres différents :
- un tronçon postérieur 61 incorporant des organes de blocage du dit étui dans sa position reculée d'injection,
- un tronçon central 69 adapté pour loger un ressort 70 de « propulsion » du dit étui vers sa position avancée de protection après usage de l'aiguille d'injection 42,
- et un tronçon antérieur 72 de protection de l'aiguille après injection, à l'intérieur duquel sont ménagés des organes de blocage irréversible du dit étui dans sa position avancée de protection après usage de l'aiguille d'injection 42.

En premier lieu, le tronçon postérieur 61 présente la forme d'une coupelle cylindrique de diamètre interne conjugué de celui de la collerette appui-doigts 32 du corps de seringue 30, adapté pour loger cette dernière ainsi qu'une bague 65 de blocage de la dite collerette dans la position reculée d'injection de l'étui de protection 60.

La paroi périphérique de cette coupelle 61 est, en outre, subdivisée en plusieurs secteurs angulaires 62 par des fentes longitudinales 63 ménagées de façon que chaque secteur forme une languette déformable 62.

De plus, une nervure périphérique 64 est ménagée à l'intérieur de cette coupelle 61, constituée par une surépaisseur de la paroi périphérique de la dite coupelle présentant longitudinalement le profil d'un triangle rectangle, de façon à définir une face antérieure radiale 64a de butée mécanique.

La bague de blocage 65 présente, quant à elle, une section longitudinale en forme de L constitué :
- d'une base 67 consistant en une couronne de diamètre externe conjugué du diamètre interne de la coupelle 61, adapté pour permettre de loger la dite bague dans la dite coupelle,
- et d'une aile périphérique 66 de hauteur adaptée pour être maintenue plaquée dans le fond de la coupelle 61 par la face de butée 64a de la nervure interne 64, la dite aile étant prolongée d'un retour en forme de crochet 68 de blocage, par encliquetage, de la bague de blocage 65 sur la collerette appui-doigts 32.

Le tronçon central 69 de l'étui de protection 60 consiste en un tube cylindrique de diamètre inférieur à celui de la coupelle 61, s'étendant à partir de la paroi de fond de la dite coupelle, de façon que la dite paroi de fond forme une face radiale annulaire de prise digitale 90 ménagée sur le pourtour du dit tronçon central.

De plus, le tronçon central 69 est lui-même prolongé par le tronçon antérieur 72 présentant le diamètre le plus petit, et il est séparé du dit tronçon antérieur par un épaulement radial 71.

Tel que précité, ce tronçon central 69 est adapté pour loger un ressort de propulsion 70 maintenu comprimé, dans la position reculée de l'étui de protection 60, entre la base 67 de la bague de blocage 65 et l'épaulement 71.

Le tronçon antérieur 72 présente, quant à lui, une longueur adaptée pour dégager le tronçon actif 42a de l'aiguille d'injection 42 dans la position reculée de l'étui de protection 60, et pour loger et masquer cette aiguille 42 dans la position avancée du dit étui de protection.

Ce tronçon antérieur 72 est, en outre, doté, tel que précité, d'organes de blocage irréversible de l'étui de protection 60 dans sa position avancée de protection après usage de l'aiguille d'injection 42.

Ces organes de blocage comprennent, en premier lieu, deux languettes déformables 73 diamétralement opposées formées, chacune, à faible distance de l'extrémité postérieure du tronçon antérieur 72, par une découpe 74 en forme de U ménagée dans la paroi du dit tronçon antérieur

Chacune de ces languettes 73 présente une surépaisseur par rapport à la paroi du tronçon antérieur 72, adaptée pour que la dite languette forme naturellement une saillie 75 délimitant une face postérieure radiale 75a à l'intérieur du dit tronçon antérieur.

Les organes de blocage comprennent, en outre, une nervure annulaire 76 formée à l'intérieur du tronçon antérieur 72 et jouxtant l'épaulement 71 ; cette nervure 76 présente le profil d'une dent asymétrique disposée de façon à présenter une face radiale antérieure de butée 76a.

Selon ce mode de réalisation, une étape préliminaire consiste à assembler l'étui de protection 60, la bague de blocage 65 et le ressort 70, de façon à comprimer le dit ressort à l'intérieur du tronçon central 69 du dit étui de protection au moyen de la bague de blocage 65 maintenue verrouillée dans la coupelle 61 par la nervure interne 64 de la dite coupelle.

Tel que notamment représenté à la figure 4, lors de l'étape suivante d'assemblage du corps de seringue 30 et de l'étui de protection pré-assemblé 60, la nervure interne 76 puis les languettes 73 du dit étui viennent, en premier lieu, en contact avec le tronçon intermédiaire 45a du capuchon protége-aiguille 43, le long duquel les dite nervure et languettes sont amenées à coulisser en se déformant radialement.

Dans la continuité du déplacement relatif de l'étui de protection 60 et du corps de seringue 30, la nervure 76 puis les languettes 73 sont ensuite amenées à coulisser le long du tronçon postérieur 44 du capuchon protége-aiguille 43, puis le long de la face périphérique de la nervure 38 du corps de seringue 30, et enfin le long du tube cylindrique 31, jusqu'à parvenir dans la position reculée d'injection du dit étui, représentée à la figure 5, dans laquelle un blocage relatif en translation réversible est obtenu par verrouillage de la collerette appui-doigts 32 au moyen de la bague de blocage 65.

Lors de ce coulissement relatif, aucun élément du corps de seringue 30 ne forme une butée orthogonale à l'axe de déplacement longitudinal de l'étui de protection 60, susceptible de constituer un obstacle s'opposant à ce coulissement relatif, de sorte que l'assemblage peut être réalisé de façon classique jusqu'à obtenir la position d'injection de l'étui de protection 60.

Par contre, après retrait du capuchon protège-aiguille 43 en vue d'une injection, la face antérieure 38b de la nervure 38 du corps de seringue 30 se trouve « démasquée », de sorte, qu'après injection et tel que représenté à la figure 6, cette face antérieure 38b forme une butée radiale pour les languettes 73 de l'étui de protection 60, provoquant un blocage irréversible de ce dernier dans sa position de protection après usage de l'aiguille d'injection 42

En outre, selon ce mode de réalisation, le déplacement de l'étui de protection 60 vers sa position avancée est obtenu automatiquement en fin de course du piston 50, du fait de la déformation, par le disque 55, de la paroi périphérique 62 de la coupelle 61, et de la libération qui s'en suit de la bague de blocage 65.

En second lieu, les figures 7-9 et 10-12 représentent deux dispositifs d'injection dont les corps de seringue présentent un diamètre d'une valeur susceptible d'autoriser à une personne d'introduire un doigt à l'intérieur de l'étui de protection.

De plus, selon les figures 7 à 9, le dispositif d'injection est du type à « déclenchement manuel » de l'étui de protection après injection, tandis que, selon les figures 10 à 12, le dispositif d'injection est du type à - « déclenchement automatique » de l'étui de protection après injection.

Par ailleurs, ces deux dispositifs d'injection présentent un grand nombre d'éléments identiques ou analogues aux éléments correspondants des dispositifs d'injection, respectivement à « déclenchement manuel » et à « déclenchement automatique », décrits ci-dessus.

Pour cette raison, les éléments de ces deux dispositifs d'injection identiques à ceux précédemment décrits sont identifiés ci-dessous par la même référence numérique et ne sont pas décrits en détail. Les éléments de ces deux dispositifs d'injection présentant une fonction similaire à celle des éléments précédemment décrits mais se différenciant de ces derniers par leur structure sont quant à eux identifiés par une même référence numérique à laquelle est adjointe le signe « ' »

Le dispositif d'injection représenté aux figures 7 à 9 comporte, en premier lieu, une seringue classique 1 identique à celle précédemment décrite et comportant donc notamment une nervure externe de blocage 7, et, associés à cette seringue 1, un piston 9 et une tige de piston 11 dotée d'un poussoir d'actionnement digital 13.

Ce dispositif d'injection comprend également un étui de protection 14' constitué d'un tube cylindrique 14a de diamètre interne adapté pour coulisser le long de la seringue 1, le dit tube se prolongeant, au niveau de son extrémité antérieure, d'un tronçon de forme tronconique 14b de section décroissante adaptée pour que la face antérieure du dit étui de protection 14' présente un diamètre non susceptible d'autoriser à une personne d'introduire un doigt à l'intérieur de cet étui de protection 14'.

Cet étui de protection 14' comporte, en outre, tel que l'étui de protection 14 :
- une nervure annulaire interne 15 de forme non rétentive, ménagée à faible distance du tronçon antérieur 14b,
- des organes de blocage internes ménagés au niveau de son extrémité postérieure, formés de deux séries de crans postérieurs 16 et antérieurs 17 délimitant une gorge annulaire de forme rétentive apte à loger la nervure annulaire 7 de la seringue 1.

Le dispositif d'injection selon les figures 7 à 9 comprend également un capuchon protége-aiguille 8' composé de deux éléments distincts, postérieur 8a et antérieur 8b, adaptés pour être accolés bout à bout et pour former, une fois assemblés, un capuchon protège-aiguille 8' identique au capuchon protége-aiguille 8 d'un seul tenant précédemment décrit.

A cet effet, l'élément antérieur 8b consiste en un élément tubulaire tronconique similaire au tronçon antérieur 21 du capuchon protége-aiguille 8, et présente donc une forme et une longueur adaptées pour loger la partie active 6a de l'aiguille d'injection 6, et pour coiffer l'embase 4.

Le second élément, postérieur, 8a de ce capuchon protége-aiguille 8'se compose quant à lui :
- d'un tronçon postérieur cylindrique 19' de faible longueur, de diamètre interne sensiblement supérieur au diamètre externe de la seringue 1, et de diamètre externe égal au diamètre externe de la nervure annulaire 7,
- d'un tronçon intermédiaire tronconique 20' de forme conjuguée de celle du tronçon antérieur tronconique 14b de l'étui de protection 14',
- et d'un raccord annulaire antérieur 22' de liaison avec l'élément antérieur 8b.

Les dimensions de ces deux éléments 8a et 8b sont adaptées pour que :
- lors du montage du capuchon 8' sur la seringue 1, l'élément antérieur 8b vienne se bloquer par un effet de coincement sur l'embase 4, et assure le blocage en translation de l'élément postérieur 8a dans une position où le dit élément postérieur est en butée contre la face antérieure radiale 7b de la nervure 7, et se profile dans le prolongement de la dite nervure,
- en fin de mise en place de l'étui de protection 14' le long de la seringue 1, l'élément postérieur 8a vienne se bloquer par un effet de coincement à l'intérieur du tronçon antérieur 14b du dit étui de protection.

Selon ce principe, et tel que notamment représenté à la figure 7, lors du coulissement de l'étui de protection 14' le long de la seringue 1, destiné à amener le dit étui de protection dans sa position reculée d'injection représentée à la figure 8, aucun élément de la seringue 1 ne forme un obstacle susceptible de s'opposer à ce coulissement, de sorte que l'assemblage peut être réalisé de façon classique.

Par la suite, l'injection peut être réalisée après le retrait, obtenu de façon classique, de l'élément antérieur 8b du capuchon protége-aiguille 8'.

Enfin, après injection et tel que représenté à la figure 9, l'élément postérieur 8a du capuchon protége-aiguille 8' est entraîné avec l'étui de protection 14' lors du coulissement de ce dernier vers sa position de protection après usage de l'aiguille d'injection, de sorte que la face antérieure 7b de la nervure 7 de la seringue 1 se trouve « démasquée » pour former une butée radiale provoquant un blocage irréversible du dit étui de protection dans la dite position de protection après usage.

Le dispositif d'injection du type à « déclenchement automatique » représenté aux figures 10 à 12, comporte un corps de seringue 30 identique à celui décrit en référence aux figures 4 à 6 constitué principalement :
- d'une seringue 31 dotée d'une collerette appui-doigts 32 au niveau de son extrémité postérieure, et prolongée, au niveau de son extrémité antérieure, par un nez tronconique 33 formant un raccord mâle à verrouillage,
- d'une embase 36 formant un raccord conique femelle 37 à verrouillage conjugué du raccord mâle 33, dans laquelle est scellé le tronçon postérieur d'une aiguille d'injection 42, et sur laquelle est ménagée une nervure externe 38 de blocage irréversible de l'étui de protection de ce dispositif d'injection.

Ce dispositif d'injection comporte, en outre, un piston 50 associé à une tige de piston 52 dotée, au niveau de son extrémité opposée, d'un poussoir 54 de diamètre sensiblement supérieur à celui de la collerette appui-doigts 32 adapté, tel que décrit plus loin, pour engendrer de façon automatique, en fin d'injection, le déclenchement du déplacement de l'étui de protection 60' vers sa position de protection après usage.

Cet étui de protection 60' comporte un tube cylindrique 72' de diamètre interne adapté pour coulisser le long de la seringue 31, à l'intérieur duquel sont ménagés des organes de blocage irréversible du dit étui dans sa position avancée de protection après usage de l'aiguille d'injection 42, le dit tube étant prolongé :
- au niveau de son extrémité postérieure, par un tronçon postérieur 61' présentant la forme d'une coupelle cylindrique de diamètre interne conjugué de celui de la collerette appui-doigts 32 de la seringue 31, adapté pour que la paroi de fond de la dite coupelle formé une face radiale annulaire de prise digitale 90' ménagée sur le pourtour du tube cylindrique 72',
- et au niveau de son extrémité antérieure, par un tronçon de forme tronconique 72a de section décroissante adaptée pour que la face antérieure de l'étui de protection 60' présente un diamètre non susceptible d'autoriser à une personne d'introduire un doigt à l'intérieur du dit étui de protection 60'.

En outre, la paroi périphérique de la coupelle 61' est subdivisée en plusieurs secteurs angulaires 62' par des fentes longitudinales ménagées de façon que chaque secteur forme une languette déformable 62'.

De plus, une nervure périphérique 64' est ménagée à l'intérieur de cette coupelle 61', constituée par une surépaisseur de la paroi périphérique de la dite coupelle présentant longitudinalement le profil d'un triangle rectangle, de façon à définir une face antérieure radiale 64a de butée mécanique.

Le tube cylindrique 72' est, en outre, doté, tel que précité, d'organes de blocage irréversible de l'étui de protection 60' dans sa position avancée de protection après usage de l'aiguille d'injection 42.

De façon similaire au dispositif d'injection décrit en référence aux figures 4 à 6, ces organes de blocage comprennent :
- deux languettes déformables 73' diamétralement opposées formées, chacune, par une découpe en forme de U ménagée dans la paroi du tube cylindrique 72',
- et une nervure annulaire 76' présentant le profil d'une dent asymétrique disposée de façon à présenter une face radiale antérieure de butée.

Le dispositif d'injection selon les figures 10 à 12 comprend également un capuchon protége-aiguille 43' composé de deux éléments distincts :
- un élément antérieur 43b consistant en un élément tubulaire tronconique de forme adaptée pour loger la partie active de l'aiguille d'injection 42, coiffer l'extrémité de l'embase 36 du corps de seringue 30 et se bloquer par un effet de coincement sur cette dernière,
- et un élément postérieur 43a de forme adaptée pour se loger dans l'étui de protection 70' et, d'une part, pour venir en butée contre la face radiale antérieure de la nervure 38 de l'embase 36, et d'autre part, pour loger un ressort 70' de « propulsion » du dit étui de protection 60' vers sa position avancée de protection après usage de l'aiguille d'injection 42.

A cet effet, cet élément postérieur 43a présente, sur sa plus grande longueur, une forme cylindrique de diamètre externe conjugué du diamètre interne du tube cylindrique 72', se prolongeant d'un tronçon 80 de forme tronconique conjuguée de celle du tronçon tronconique 72a de l'étui de protection 70'.

La portion cylindrique de cet élément postérieur 43a présente, en outre, un diamètre interne adapté pour délimiter, autour de l'embase 36, un espace annulaire apte à loger un ressort de propulsion 70' conçu pour être maintenu comprimé dans ce logement entre la face radiale antérieure de la nervure 38 de l'embase 36 et une face radiale formée à l'intérieur du tronçon tronconique 80 du dit élément postérieur.

Enfin, et tel que représenté à la figure 10, les éléments postérieur 43a et antérieur 43b sont adaptés pour s'accoupler dans le prolongement l'un de l'autre, dans une position où l'élément antérieur 43b assure un blocage en translation de l'élément postérieur 43a à l'encontre de l'effort exercé sur ce dernier par le ressort de propulsion 70'.

Il est à noter que ce blocage en translation résulte de l'effet de coincement résultant du montage en force de l'élément antérieur 43b sur l'embase 36. A des fins de sécurité, ce blocage peut toutefois être également assuré en dotant l'embase 36 et l'élément antérieur 43b d'organes d'encliquetage complémentaires de tout type connu en soi.

Selon ce mode de réalisation et en premier lieu, le capuchon protége-aiguille 43' dans lequel est positionné le ressort de propulsion 70' est monté sur le corps de seringue 30. Une fois ce montage effectué, l'élément antérieur 43b assure le blocage en translation de l'élément postérieur 43a dans une position où le dit élément postérieur loge le ressort de propulsion 70' maintenu comprimé, et vient buter contre la face antérieure radiale de la nervure 38 et se profile dans le prolongement de cette dernière.

Une fois cette étape préliminaire effectuée, l'étui de protection 70' est monté autour du corps de seringue 30. Lors de ce montage, obtenu en faisant coulisser l'étui de protection 70' le long du corps de seringue 30 en vue de l'amener dans sa position reculée d'injection représentée à la figure 11, aucun élément ne forme un obstacle susceptible de s'opposer à ce coulissement, de sorte que l'assemblage peut être réalisé de façon classique.

Enfin, une fois l'étui de protection 70' parvenu dans sa position reculée d'injection, un blocage relatif en translation réversible est obtenu par verrouillage de la collerette appui-doigts 32 à l'intérieur de la coupelle 61'.

Dans cette position, en outre, l'élément postérieur 43a du capuchon protége-aiguille 43'se trouve logé et bloqué en translation à l'intérieur de l'étui de protection 70', de sorte que l'élément antérieur 43b de ce capuchon peut être retiré, en vue d'une injection, sans entraîner un quelconque déplacement du dit étui de protection.

Par la suite, en fin d'injection, le déplacement de l'étui de protection 60' vers sa position avancée est déclenché automatiquement grâce au ressort de propulsion 70', en fin de course du piston 50, du fait de la déformation radiale, par le poussoir 54, des nervures 64' de la coupelle 61', et de la libération qui s'en suit du dit étui de protection

Lors de ce déplacement, en outre, l'élément postérieur 43a du capuchon protége-aiguille 43', sollicité par le ressort de propulsion 70', est entraîné avec l'étui de protection 70', de sorte que la face antérieure de la nervure 38 du corps de seringue 30 se trouve « démasquée » pour former une butée radiale provoquant un blocage irréversible du dit étui de protection dans sa position de protection après usage de l'aiguille d'injection 42.

## Revendications

1. Dispositif à usage unique d'injection d'une dose de fluide médicamenteux, comprenant:
- un corps de seringue (1 ; 30) comportant, d'une part, une embase (4 ; 36) portant une aiguille d'injection (6 ; 42) dotée d'une partie active d'injection (6a ; 42a) dans le prolongement de la dite embase, et, d'autre part, à l'opposé de cette embase (4 ; 36), des organes de prise digitale (3 ; 32) d'un seul tenant avec le dit corps de seringue (1 ; 30),
le dit capuchon protège-aiguille (8 ; 43 ; 8' ; 43') comportant longitudinalement un tronçon antérieur (21 ; 45 ; 8b ; 43b) de longueur adaptée pour loger au moins partiellement la partie active (6a ; 42a) de l'aiguille d'injection (6 : 42), et un tronçon postérieur (19 ; 44 ; 19' ; 43a) relié au dit tronçon antérieur par un tronçon intermédiaire (20 ; 45a ; 20' ; 80) de forme tronconique s'évasant en direction du dit tronçon postérieur,
- un capuchon protège-aiguille (8 ; 43 ; 8' ; 43') de forme adaptée pour loger la partie active (6a ; 42a) de l'aiguille d'injection (6 : 42), et coiffer l'embase (4 ; 36) du corps de seringue (1 ; 30), [ - ]
- un étui tubulaire (14 ; 60 ; 14' ; 60') de protection après usage de l'aiguille d'injection (6 ; 42), de section interne adaptée pour coulisser extérieurement le long du corps de seringue (1 ; 30),
- les dits corps de seringue et étui de protection étant dotés d'organes de blocage relatif en translation consistant en :
. des premiers organes de blocage (7, 15 ; 32, 62-65 ; 62', 64') disposés et adaptés pour bloquer l'étui de protection (14 ; 60 ; 14' ; 60') dans une position reculée, dite position d'injection, dans laquelle le dit étui d'injection se trouve escamoté longitudinalement par rapport à la partie active (6a ; 42a) de l'aiguille d'injection (6 ; 42),
. et des seconds organes de blocage (7, 16, 17 ; 38, 73-76 ; 38, 73', 76') ménagés sur le corps de seringue (1 ; 30) et à l'intérieur de l'étui de protection (14 ; 60; 14' ; 60'), adaptés pour bloquer de facon irréversible l'étui de protection dans une position avancée de protection après usage de l'aiguille d'injection (6 ; 42), dans laquelle le dit étui de protection s'étend partiellement dans le prolongement du corps de seringue (1 ; 30) de façon à loger la partie active (6a ; 42a) de la dite aiguille d'injection,
. les dits seconds organes de blocage menagés sur le corps de seringue (1 ; 30) présentant une section externe adaptée pour coulisser à l'intérieur de l'étui de protection (14 ; 60 ; 14' ; 60'), et formant, sur le pourtour du dit corps de seringue, deux faces radiales annulaires, dites face de butée postérieure (7a ; 38a) et face de butée antérieure (7b ; 38b),
. et les dits seconds organes de blocage ménagés a l'intérieur de l'étui de protection (14 ; 60 ; 14' ; 60') formant deux faces radiales de butée (16b, 17b ; 75a, 76a) s'étendant en regard l'une de l'autre, menagées de façon à coopérer chacune avec une des faces de butée radiale (7a, 7b ; 38a, 38b) formées sur le pourtour du corps de seringue (1 ; 30),
le dit dispositif d'injection à usage unique étant **caractérisé en ce que :**
- le dit capuchon protège-aiguille présente une longueur totale adaptée pour que l'extrémité postérieure du tronçon postérieur (19 ; 44 ; 19' ; 43a) vienne buter contre la face de butée antérieure (7b ; 38b) des seconds organes de blocage ménagés sur le corps de seringue (1 ; 30),
- et le dit tronçon postérieur présente un contour conjugué de celui de la face de butée antérieure (7b ; 38b) précitée, de façon à se profiler extérieurement avec cette dernière, et à la masquer.

2. Dispositif d'injection selon la revendication 1 doté d'un étui de protection (14 ; 14') à déclenchement manuel après injection, **caractérisé en ce que** les premiers organes de blocage relatif en translation (7, 15) comprennent une nervure interne (15) de section non rétentive ménagée au niveau d'une portion de tronçon antérieur du dit étui de protection, et adaptée pour coopérer avec la face de butée postérieure (7a) des seconds organes de blocage (7) ménagés sur le corps de seringue (1).

3. Dispositif d'injection selon l'une des revendications 1 ou 2, **caractérisé en ce que** le corps de seringue (30) comporte un récipient tubulaire (31) prolongé d'un raccord conique mâle (33), et une embase (36) formant un raccord conique femelle conjugué (37), et sur laquelle sont ménagés les seconds organes de blocage relatif en translation (38).

4. Dispositif d'injection selon la revendication 2 **caractérisé en ce que** le corps de seringue (1) comporte, d'un seul tenant, un récipient tubulaire (2) prolongé d'une embase (4) porte-aiguille d'injection (6), et sur lequel sont ménagés, sensiblement en retrait longitudinal par rapport à la dite embase, les seconds organes de blocage relatif en translation (7).

5. Dispositif d'injection selon l'une des revendications 3 ou 4 , **caractérisé en ce que** les seconds organes de blocage (7 ; 38) ménagés sur le corps de seringue (1 ; 30) consistent en une nervure annulaire (7 ; 38) dotée d'une paroi périphérique circulaire de diamètre adapté pour former un appui de guidage du coulissement de l'étui de protection (14 ; 60 ; 14' ; 60'), et de deux parois frontales radiales formant les faces postérieure (7a ; 38a) et antérieure (7b ; 38b) de butée.

6. Dispositif d'injection selon l'une des revendications précédentes **caractérisé en ce que :**
- l'étui tubulaire (14' ; 60') comporte un tronçon antérieur (14b ; 72a) de section interne décroissant longitudinalement,
- le capuchon protége-aiguille (8' ; 43') se compose de deux éléments distincts (8a, 8b ; 43a, 43b) aptes à être assemblés dans le - prolongement l'un de l'autre:
■ un élément antérieur (8b ; 43b) formant le tronçon antérieur du dit capuchon protège-aiguille, et adapté pour coiffer l'embase (4 ; 36) du corps de seringue (1 ; 30),
■ et un élément postérieur (8a ; 43a) formant les tronçons intermédiaire (20' ; 80) et postérieur (19' ; 43a) du capuchon protège-aiguille (8' ; 43'), et adapté pour être logé à l'intérieur de l'étui de protection (14' ; 60') dans la position reculée d'injection de ce dernier,
■ l'élément postérieur (8a ; 43a) étant adapté, après retrait de l'élément antérieur (8b ; 43b), pour être entraîné avec l'étui de protection (14' ; 60') lors du déplacement de ce dernier vers sa position de protection après usage de l'aiguille d'injection (6 ; 42).

7. Dispositif d'injection selon la revendication 6 **caractérisé en ce que** l'élément postérieur (8a) du capuchon protége-aiguille (8') est conformé de façon à constituer un tronçon intermédiaire adapté pour se bloquer par un effet de coincement à l'intérieur du tronçon antérieur (14b) de l'étui de protection (14'), dans la position d'injection de ce dernier.

8. Dispositif d'injection selon la revendication 6 **caractérisé en ce que** l'élément postérieur (43a) du capuchon protége-aiguille (43) délimite, autour de l'embase (36) du corps de seringue (30), un volume annulaire apte à loger dans son état comprimé un ressort de propulsion (70') adapté pour entraîner, après déclenchement, un déplacement simultané du dit élément postérieur et de l'étui de protection (60').

## Claims

1. Disposable device for injecting a dose of medicinal fluid, comprising:
- a syringe body (1; 30) comprising, on the one hand, a seat (4; 36) which carries an injection needle (6; 42) which is equipped with an active injection part (6a; 42a) in the extension of said seat and, on the other hand, opposite this seat (4; 36), members for gripping by the fingers (3; 32) integral with said syringe body (1; 30),
- a needle-protection cap (8; 43; 8'; 43') of a shape which is adapted to house the active part (6a; 42a) of the injection needle (6; 42) and to cover the seat (4; 36) of the syringe body (1; 30), said needle-protection cap (8; 43; 8'; 43') comprising longitudinally a front section (21; 45; 8b; 43b) of a length which is adapted to house at least partially the active part (6a; 42a) of the injection needle (6; 42), and a rear section (19; 44; 19'; 43a) which is connected to said front section by an intermediate section (20; 45a; 20'; 80) of a tapered form which is flared in the direction of said rear section,
- a tubular case (14, 60; 14'; 60') for protection after use of the injection needle (6; 42), with an internal section which is adapted to slide on the exterior along the syringe body (1; 30),
- said syringe body and protective case being equipped with members for relative locking in translation, comprising:
• first locking members (7, 15; 32, 62-65; 62', 64') which are disposed and adapted to lock the protective case (14; 60; 14'; 60') in a retracted position, termed injection position, in which said injection case is situated retracted longitudinally relative to the active part (6a; 42a) of the injection needle (6; 42),
• and second locking members (7, 16, 17; 38, 73-76; 38, 73', 76') which are provided on the syringe body (1; 30) and inside the protective case (14; 60; 14'; 60') and adapted to lock the protective case irreversibly in a forward protective position after use of the injection needle (6; 42), in which position said protective case extends partially into the extension of the syringe body (1; 30) so as to house the active part (6a; 42a) of said injection needle,
• said second locking members which are provided on the syringe body (1; 30) having an external section which is adapted to slide inside the protective case (14; 60; 14'; 60') and forming, on the circumference of said syringe body, two annular radial faces, termed rear limit stop face (7a; 38a) and front limit stop face (7b; 38b),
• and said second locking members which are provided inside the protective case (14; 60; 14'; 60') forming two radial limit stop faces (16b, 17b; 75a, 76a) which extend opposite each other and are provided so as to cooperate each with one of the radial limit stop faces (7a, 7b; 38a, 38b) which are formed on the circumference of the syringe body (1; 30),
said disposable injection device being **characterised in that:**
- said needle-protection cap has a total length which is adapted such that the rear end of the rear section (19; 44; 19'; 43a) comes to abut against the front limit stop face (7b; 38b) of the second locking means which are provided on the syringe body (1; 30),
- and said rear section has a contour which is conjugate with that of the aforementioned front limit stop face (7b; 38b) so as to form an exterior profile with the latter and to mask it.

2. Injection device according to claim 1, equipped with a protective case (14; 14') with manual release after injection, **characterised in that** the first members for relative locking in translation (7, 15) comprise an internal rib (15) with a non-retentive section which is provided at the level of a front section part of said protective case and adapted to cooperate with the rear limit stop face (7a) of the second locking members (7) which are provided on the syringe body (1).

3. Injection device according to one of the claims 1 or 2, **characterised in that** the syringe body (30) comprises a tubular receptacle (31) extended by a conical male connection (33), and a seat (36) forming a conjugate conical female connection (37), and on which there are provided the second members for relative locking in translation (38).

4. Injection device according to claim 2, **characterised in that** the syringe body (1) comprises, integrally, a tubular receptacle (2) extended by a seat (4) carrying the injection needle (6), and on which there are provided, essentially retracted longitudinally relative to said seat, the second members for relative locking in translation (7).

5. Injection device according to one of the claims 3 or 4, **characterised in that** the second locking members (7; 38) which are provided on the syringe body (1; 30) comprise an annular rib (7; 38) which is equipped with a circular peripheral wall of a diameter adapted to form a support stop for guiding the sliding of the protective case (14; 60; 14'; 60') and two radial front walls forming the rear (7a; 38a) and front (7b; 38b) limit stop faces.

6. Injection device according to one of the preceding claims, **characterised in that**
- the tubular case (14'; 60') comprises a front section (14b; 72a) of an internal section which decreases longitudinally,
- the needle-protection cap (8'; 43') is composed of two separate elements (8a, 8b; 43a, 43b) which are able to be assembled in the extension one to the other:
• a front element (8b; 43b) forming the front section of said needle-protection cap and adapted to cover the seat (4; 36) of the syringe body (1; 30),
• and a rear element (8a; 43a) forming the intermediate (20'; 80) and rear (19'; 43a) sections of the needle-protection cap (8'; 43') and adapted to be housed inside the protective case (14'; 60') in the retracted injection position of the latter,
• the rear element (8a; 43a) being adapted, after retraction of the front element (8b; 43b), to be moved with the protective case (14'; 60') during displacement of the latter towards the protective position thereof after use of the injection needle (6; 42).

7. Injection device according to claim 6, **characterised in that** the rear element (8a) of the needle-protection cap (8') is shaped so as to form an intermediate section which is adapted to be locked by a clamping effect inside the front section (14b) of the protective case (14') in the injection position of the latter.

8. Injection device according to claim 6, **characterised in that** the rear element (43a) of the needle-protection cap (43) delimits, around the seat (36) of the syringe body (30), an annular volume which is able to house in the compressed state thereof a propulsion spring (70') which is adapted to induce, after release, a simultaneous displacement of said rear element and of the protective case (60').

## Patentansprüche

1. Vorrichtung zur einmaligen Anwendung einer Injektion einer Dosis eines medikamentösen Fluids, mit:
- einem Spritzenkörper (1; 30), der einerseits ein Unterteil (4; 36) aufweist, das eine Injektionsnadel (6; 42) trägt, die mit einem aktiven Injektionsteil (6a; 42a) in der Verlängerung des Unterteils versehen ist, und andererseits, gegenüber diesem Unterteil (4; 36), Fingergriffmittel (3; 32) aufweist, die mit dem Spritzenkörper (1; 30) integral sind,
- einer Nadelschutzkappe (8; 43; 8'; 43') mit einer Form, die zum Unterbringen des aktiven Teils (6a; 42a) der Injektionsnadel (6; 42) und zum Aufsetzen auf das Unterteil (4; 36) des Spritzenkörpers (1; 30) angepasst ist, wobei die Nadelschutzkappe (8; 43; 8'; 43') der Länge nach ein vorderes Teilstück (21; 45; 8b; 43b) aufweist mit einer Länge, die dazu angepasst ist, um den aktiven Teil (6a; 42a) der Injektionsnadel (6; 42) zumindest teilweise unterzubringen, und ein hinteres Teilstück (19; 44; 19'; 43a) aufweist, das mit dem vorderen Teilstück über ein mittleres Teilstück (20; 45a; 20', 80) verbunden ist, mit einer kegeligen Form, die sich in Richtung des hinteren Teilstücks aufweitet,
- einer rohrförmigen Schutzhülse (14; 60; 14'; 60') zum Schutz nach Verwendung der Injektionsnadel (6; 42) mit einem inneren Querschnitt, der angepasst ist, um außen längs des Spritzenkörpers (1; 30) verschoben zu werden,
- wobei die Körper der Spritze und der Schutzhülse mit Sperrmitteln zum relativen Sperren gegen Verschiebung versehen sind, bestehend aus:
■ ersten Sperrmitteln (7; 15; 32, 62-65; 62', 64'), die angeordnet und angepasst sind, um die Schutzhülse (14; 60; 14'; 60') in einer zurückgeschobenen Position, der sogenannten Injektionsposition, in der sich die Injektionshülse in Bezug auf den aktiven Teil (6a; 42a) der Injektionsnadel (6; 42) der Länge nach eingezogen befindet, zu blockieren,
■ und zweiten Sperrmitteln (7, 16, 17; 38, 73-76; 38, 73', 76'), die auf dem Spritzenkörper (1; 30) und am Inneren der Schutzhülse (14; 60; 14'; 60') vorgesehen sind, und dazu angepasst sind, um auf irreversible Weise die Schutzhülse nach der Verwendung der Injektionsnadel (6; 42) in einer vorgeschobenen Schutzposition, in der sich die Schutzhülse teilweise in die Verlängerung des Spritzenkörpers (1; 30) erstreckt, zu blockieren, so dass der aktive Teil (6a; 42a) der Injektionsnadel untergebracht wird,
■ wobei die zweiten Sperrmittel, die auf dem Spritzenkörper (1; 30) vorgesehen sind, einen äußeren Querschnitt aufweisen, der dazu angepasst ist, um zum Inneren der Schutzhülse (14; 60; 14'; 60') verschoben zu werden, und um auf dem Umfang des Spritzenkörpers zwei radiale ringförmige Seiten, die sogenannte Seite des hinteren Anschlags (7a; 38a) und die sogenannte Seite des vorderen Anschlags (7b; 38b), zu bilden,
■ und wobei die zweiten Sperrmittel, die am Inneren der Schutzhülse (14; 60; 14'; 60') vorgesehen sind, zwei radiale Anschlagseiten (16b, 17b; 75a, 76a) bilden, die sich zueinander erstrecken und auf eine Weise angeordnet sind, damit jede mit einer der radialen Anschlagseiten (7a, 7b; 38a, 38b) zusammenwirkt, die auf dem Umfang des Spritzenkörpers (1; 30) gebildet sind,
wobei die Injektionsvorrichtung zur einmaligen Verwendung **dadurch gekennzeichnet ist, dass**:
- die Nadelschutzkappe eine Gesamtlänge aufweist, die angepasst ist, damit das hintere Ende des hinteren Teilstücks (19; 44; 19', 43a) gerade gegen die Seite des vorderen Anschlags (7b; 38b) der zweiten Sperrmittel, die auf dem Spritzenkörper (1; 30) vorgesehen sind, anstößt,
- und das hintere Teilstück eine Kontur aufweist, die derjenigen der Seite des vorgenannten vorderen Anschlags (7b; 38b) auf eine Weise entspricht, um sich mit dieser letzteren außen im Profil abzuheben und sie abzudecken.

2. Vorrichtung zur Injektion nach Anspruch 1,
die mit einer Schutzhülse (14; 14') zur manuellen Auslösung nach einer Injektion versehen ist,
**dadurch gekennzeichnet,**
**dass** die ersten Sperrmittel (7, 15) zum relativen Sperren gegen Verschiebung eine innere Rippe (15) mit einem nicht wasserspeichernden Querschnitt aufweisen, die auf Höhe eines Bereiches des vorderen Abschnitts der Schutzhülse angeordnet ist und angepasst ist, um mit der Seite des hinteren Anschlags (7a) der zweiten Sperrmittel (7), die auf dem Spritzenkörper (1) angeordnet sind, zusammenzuwirken.

3. Injektionsvorrichtung nach einem der Ansprüche 1 oder 2,
**dadurch gekennzeichnet,**
**dass** der Spritzenkörper (30) einen rohrförmigen Behälter (31) aufweist, der um einen männlichen konischen Anschluss (33) verlängert ist, und ein Unterteil (36) aufweist, das einen zugeordneten konischen weiblichen Anschluss (37) bildet, und auf dem die zweiten Sperrmittel (38) zum relativen Sperren gegen Verschiebung vorgesehen sind.

4. Injektionsvorrichtung nach Anspruch 2,
**dadurch gekennzeichnet,**
**dass** der Spritzenkörper (1) integral einen rohrförmigen Behälter (2) aufweist, der um ein Unterteil (4) des Injektionsnadelträgers (6) verlängert ist und auf dem, längs zum Unterteil merklich eingerückt, die zweiten Sperrmittel (7) zum relativen Sperren gegen Verschiebung angeordnet sind.

5. Injektionsvorrichtung nach einem der Ansprüche 3 oder 4,
**dadurch gekennzeichnet,**
**dass** die zweiten Sperrmittel (7; 38), die auf dem Spritzenkörper (1; 30) angeordnet sind, aus einer ringförmigen Rippe (7; 38), die mit einer kreisförmigen peripheren Wandung versehen ist, deren Durchmesser angepasst ist, um eine Stütze zur Führung der Verschiebung der Schutzhülse (14; 60; 14'; 60') zu bilden, und zwei radialen stirnseitigen Wandungen bestehen, welche die Seite des hinteren (7a; 38a) und des vorderen (7b; 38b) Anschlags bilden.

6. Injektionsvorrichtung nach einem der vorstehenden Ansprüche,
**dadurch gekennzeichnet, dass**
- die rohrartige Hülse (14'; 60') ein vorderes Teilstück (14b; 72a) mit einem internen Querschnitt aufweist, der sich in Längsrichtung verengt,
- sich die Nadelschutzkappe (8'; 43') aus zwei verschiedenen Elementen (8a, 8b; 43a, 43b) zusammensetzt, die geeignet sind, in der Verlängerung miteinander zusammengesetzt zu werden;
• ein vorderes Element (8b; 43b), das das vordere Teilstück der Nadelschutzkappe bildet, und angepasst ist, um auf das Unterteil (4; 36) des Spritzenkörpers (1; 30) aufgesetzt zu werden,
• und ein hinteres Element (8a; 43a), das das mittlere (20'; 80) und hintere (19'; 43a) Teilstück der Nadelschutzkappe (8'; 43) bildet und angepasst ist, um im Inneren der Schutzhülse (14'; 60') in der zurückgeschobenen Injektionsposition dieser letzteren untergebracht zu sein,
• das hintere Element (8a; 43a) angepasst ist, um nach dem Rückzug des vorderen Elementes (8b; 43b) mit der Schutzhülse (14'; 60') während der Versetzung dieser letzteren in ihre Schutzposition nach Verwendung der Injektionsnadel (6; 42) mitgenommen zu werden.

7. Injektionsvorrichtung nach Anspruch 6,
**dadurch gekennzeichnet,**
**dass** das hintere Element (8a) der Nadelschutzkappe (8') auf eine Weise gebildet ist, um ein mittleres Teilstück zu bilden, das angepasst ist, um sich durch einen Klemmeffekt im Inneren des vorderen Teilstücks (14b) der Schutzhülse (14') in der Injektionsposition dieser letzteren zu blockieren.

8. Injektionsvorrichtung nach Anspruch 6,
**dadurch gekennzeichnet,**
**dass** das hintere Element (43a) der Nadelschutzkappe (43) rings um das Unterteil (36) des Spritzenkörpers (30) herum ein ringförmiges Volumen begrenzt, das geeignet ist, im komprimierten Zustand eine Antriebsfeder (70') zu beherbergen, die angepasst ist, um nach Auslösung eine gleichzeitige Versetzung des hinteren Elementes und der Schutzhülse (60') anzutreiben.
